# EUROPEAN PATENT APPLICATION

(11) **EP 1 149 816 A1**
(43) Date of publication of application: **31.10.2001**
(21) Application number: 00978064.4
(22) Date of filing: 01.12.2000
(51) Int. Cl.: C07C 39/16, C07C 37/84, C07C 37/82

(54) **HIGH-QUALITY BISPHENOL A AND PROCESS FOR PRODUCING THE SAME**

(30) Priority: 03.12.1999 JP 34409499; 03.12.1999 JP 34409599; 03.12.1999 JP 34409699; 03.12.1999 JP 34409799; 03.12.1999 JP 34409899; 31.05.2000 JP 2000161483; 31.05.2000 JP 2000161484; 31.05.2000 JP 2000161485
(71) Applicant: Mitsui Chemicals, Inc., Tokyo 100-6070 (JP)
(72) Inventor: Nakamura, Hideaki, Takaishi-shi, Osaka 592-8501 (JP); Inomata, Masamitsu, Takaishi-shi, Osaka 592-8501 (JP); Miura, Toshizumi, Takaishi-shi, Osaka 592-8501 (JP)
(74) Representative: Kirkham, Nicholas Andrew
(86) International application number: JP0008534
(87) International publication number: WO0140156

(57) **Abstract**

The present invention is a high-quality bisphenol A wherein the content of sodium is not more than 80 ppb and the content of each element of the group A consisting of iron, nickel, chromium, manganese, lead, and molybdenum is not more than 50 ppb, or a high-quality bisphenol A containing an organic compound such as 9,9'-dimethylxanthene, 2-(4-hydroxyphenyl)-2,4,4-trimethylchroman, 2,2,4-trimethylchromene, etc., in an amount of lower than a specific amount, and a preparation process of these high-quality bisphenol A. When an aromatic polycarbonate is prepared using the high-quality bisphenol of the invention, an aromatic polycarbonate which is not colored at the preparation thereof and molding the polycarbonate.

## Description

### TECHNICAL FIELD

The present invention relates to high-quality bisphenol A and to a preparation process thereof, and more specifically to a bisphenol A which is a raw material for producing an aromatic polycarbonate having a good hue and also to a preparation process thereof.

### BACKGROUND OF THE INVENTION

Bisphenol A [2,2-bis(A-hydroxyphenyl)propane] is usually prepared by reacting an excessive amount of phenol and acetone in the existence of a homogeneous acid or a solid acid catalyst, cooling the reaction mixture obtained, and after separating equimolar adducts of bisphenol A and phenol from the reaction mixture, further removing therefrom phenol.

The bisphenol A is used as a raw material for producing various polymers, and recently, particularly, the demand for an aromatic polycarbonate excellent in the shock resistance and the transparency has been increased, and bisphenol A giving an aromatic polycarbonate having less coloring has been demanded.

Because an aromatic polycarbonate is excellent in the shock resistance and the transparency, the polymer is frequently used as optical uses, and particularly for optical disk bases or substrates. For optical disks, by increasing the recording density and increasing the capacity, the demand for the reliability for recording and reproducing has been increased and the demand for the hue and the transparency giving influences on the reliability has been more and more increased.

As a preparation process of an aromatic polycarbonate, a phosgene process and a transesterification process are mainly known. Because the transesterification process does not have problems of not using a toxic substance such as phosgene, not by-producing a corrosive substance as compared with the phosgene process, the ester exchange process has recently been given attention. However, the aromatic polycarbonate obtained by the transesterification process has a problem that the product obtained is generally liable to be colored because the polymerization is carried out at a high temperature, the improvement of the preparation process of an aromatic polycarbonate and also further improvement of the quality of bisphenol A have been required.

As a process relating to bisphenol A itself causing no coloring and being excellent in the hue, there are a process for decoloring bisphenol A by an acid treatment with thioglycollic acid, etc., (Japanese Patent Publication No. 43937/1982), a process for preparing bisphenol A using purified phenol obtained by distillation treating after contacting industrial phenol with a strong acid-type ion-exchange resin, as washing phenol in the case of washing the crystal adduct of bisphenol A and phenol using phenol (Japanese Patent Laid-Open No. 39239/1993), a process for purifying bisphenol A by the addition of hypophosphorous acid and a heat treatment (Japanese Patent Laid-Open No. 238043/1995), a process of color-stabilizing bisphenol A by adding α-hydroxypolycarboxylic acid to the bisphenol as an iron ion blocking agent (Japanese Patent Laid-Open No. 309796/1995), etc.

However, even when the bisphenol A showing a good hue obtained by the above-described processes is used, the occurrence of coloring is yet observed in the polymer moldings obtained from the bisphenol A, and thus, such a bisphenol A is insufficient as the raw material for polymers severe in the requirement of hue, such as aromatic polycarbonate for optical use.

Also, for preventing the occurrence of coloring of polymers at the preparation and molding thereof, as bisphenol A as a raw material for polymers, there are proposed bisphenols for polymer raw materials, wherein 4,4'-bisphenols each containing at least one kind of the phenolic compound selected from 2,4'-bisphenols, trisphenols, and alkyl-substituted 4,4'-bisphenols in the range of from 500 to 10,000 ppm by weight as the sum total (Japanese Patent Laid-Open No. 245465/1996). However, even by using such a bisphenol A, in the case of preparing an aromatic polycarbonate, the coloring prevention effect is insufficient.

As described above, in the bisphenol A obtained by the processes of prior art, there is a problem in the quality as the raw material of polymers for optical use and there is a difficulty in the use of such bisphenol A.

Accordingly, the present invention provides a bisphenol A which does not coloring at preparing an aromatic polycarbonate and in the case of molding the aromatic polycarbonate obtained.

### DISCLOSURE OF THE INVENTION

As the result of various investigations for solving the above-described problems, the present inventors have found that when specific metals or organic substances exist in bisphenol A in the amounts of more than definite amounts, coloring occurs at the preparation of an aromatic polycarbonate from the bisphenol A and also coloring is liable to occur in the case of molding the aromatic polycarbonate obtained, and have accomplished the present invention.

That is, an aspect of the present invention is a high-quality bisphenol A selected from the following groups (a) to (g);
(a) A high-quality bisphenol A wherein content of sodium is not more than 80 ppb and the content of each element of group A consisting of iron, nickel, chromium, manganese, lead, and molybdenum is not more than 50 ppb.
(b) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 15.5 minutes to 24 minutes is not more than 2.0 × 10⁻³ as the rate to the absorption peak area of the bisphenol A.
(c) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 22 minutes to 24 minutes is not more than 50 ppm to the absorption peak area of the bisphenol A.
(d) A high-quality bisphenol A wherein the sum total of the contents of the 1-naphthol derivatives represented by following formula (1) is not more than 200 ppm to the bisphenol A; 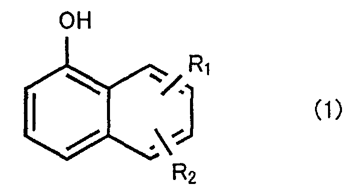 wherein R₁ and R₂ each represents a methyl group, an ethyl group, an n-propyl group, an isopropyl group, or an isopropenyl group each bonded to the 2 to 8 position of the 1-naphthol.
(e) A high-quality bisphenol A wherein the content of 9,9'-dimethylxanthene is not more than 2 ppm.
(f) A high-quality bisphenol A wherein the content of 2-(4-hydroxyphenyl)-2,4,4-trimethylchroman is not more than 50 ppm.
(g) A high-quality bisphenol A wherein the content of 2,2,4-trimethylchromene is not more than 5 ppm.
   Also, other aspect of the invention is, in a process for preparing bisphenol A by reacting phenol and acetone, the process of preparing a high-quality bisphenol A in which the content of sodium is not more than 80 ppb, and the content of each element of group A consisting of iron, nickel, chromium, manganese, lead, and molybdenum is not more 50 ppb, which comprises contacting the reaction mixture obtained by the reaction of phenol and acetone with an adsorbent.
   Furthermore, another aspect of the invention is, in a process of forming bisphenol A by reacting phenol and acetone in a reactor and separating equimolar adducts of the bisphenol A and phenol, and a mother liquor containing phenol from the reaction mixture obtained, the process for preparing a high-quality bisphenol A in which the content of sodium is not more than 80 ppb, and the content of each element of group A consisting of iron, nickel, chromium, manganese, lead, and molybdenum is not more than 50 ppb, which comprises after contacting the mother liquor with an adsorbent, the mother liquor is recycled to the reactor.

### BEST MODE FOR CARRYING OUT THE INVENTION

Bisphenol A can be generally obtained by a dehydration condensation reaction of acetone and excessive phenol in the existence of an acid catalyst. There is no particular restriction on the catalyst of preparing the bisphenol A of the present invention, and each one of a homogeneous acid and a solid acid may be used.

In the process of using a homogeneous acid, as the catalyst, hydrogen chloride, sulfuric acid, etc., is generally used but hydrogen chloride is preferably used because in this case, the hydrogen chloride can be easily separated from the reaction mixture. In the case of using hydrogen chloride as the catalyst, after the reaction, the catalyst is separated from the reaction mixture, it is preferred that the chloride ion concentration after the catalyst separation step is not more than 10 ppm, and particularly not more than 1 ppm. When the chloride ion concentration is not more than 10 ppm, the corrosion of the apparatus after catalyst separation step is less and the amount of each metal component entering the bisphenol A becomes less. As a method of separating the catalyst, there are a method of removing the catalyst by distillation, a method of removing the catalyst by adsorption using a anion-exchange resin, a method of removing the catalyst by adsorption using a anion-exchange resin after applying a distillation, etc., and any method may be used but the method of removing the catalyst by adsorption using a anion-exchange resin after applying a distillation is preferably used.

There is no particular restriction on the anion-exchange resin used for removing the catalyst by adsorption, each of a strong basic ion-exchange resin and a weak basic ion-exchange resin can be used, but the weak basic ion-exchange resin is preferably used because of the easiness of the regeneration of the ion-exchange resin. More preferably, a pyridyl group type weak basic ion-exchange resin having a high heat resistance is used.

Also, in the process of using a solid acid, a sulfonic acid-base cation-exchange resin is generally used as the catalyst. Furthermore, for improving the catalytic activity of the sulfonic acid-base cation-exchange resin, a process of existing a mercapto group-containing compound in the reaction system together with the cation-exchange resin. As a method of coexisting the cation-exchange resin and the mercapto group-containing compound, there are a method of bonding a part of the sulfonic acid group of the cation-exchange resin and the mercapto group-containing compound by an ionic bond or by a covalent bond and a method of continuously supplying a mercapto group-containing compound, which does not form a chemical bond with the cation-change resin, to a reactor packed with the sulfonic acid-base cation-exchange resin together with reaction raw materials, and each of these methods may be used.

As other examples of the solid acid catalyst, there is an organic high molecule siloxane having a sulfonic acid-containing hydrocarbon group and a mercapto group-containing hydrocarbon group together. Examples of the catalyst are organic high molecular siloxanes each having a structure wherein, in a silica matrix made of a siloxane bond, a hydrocarbon group partially having a sulfonic acid group and a hydrocarbon group having a mercapto group are directly bonded to the silicon atoms in the silica matrix by a carbon-silicon bond as described in Japanese Patent Laid-Open Nos. 208545/1996, 110989/1997, and 225638/1998.

About the reaction mixture obtained using each of the catalysts described above, the catalyst is separated therefrom, after, if necessary, removing a part of water formed by the reaction, unreacted acetone, and unreacted phenol, the reaction product is cooled and bisphenol A is separated as the form of adducts. Furthermore, from the equimolar addition product of bisphenol A and phenol, which are the adducts, phenol is removed and bisphenol A is isolated.

The bisphenol A thus obtained contains various inorganic elements and organic compounds as impurities. When the bisphenol A is used as the raw material for preparing an aromatic polycarbonate, coloring occurs at the preparation of the aromatic polycarbonate and also at molding the aromatic polycarbonate obtained, coloring is liable to occur. Accordingly, to obtain an aromatic polycarbonate which is not colored at the preparation of the aromatic polycarbonate and does not cause coloring in the case of molding the aromatic polycarbonate, it is necessary to reduce the contents of specific inorganic elements and organic compounds contained, as impurities, in the bisphenol A used as the raw material to lower than definite amounts.

The high-quality bisphenol A of the invention is selected from the following groups (a) to (g).
(a) A high-quality bisphenol A wherein content of sodium is not more than 80 ppb and the content of each element of group A consisting of iron, nickel, chromium, manganese, lead, and molybdenum is not more than 50 ppb.
(b) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 15.5 minutes to 24 minutes is not more than 2.0 × 10⁻³ as the rate to the absorption peak area of the bisphenol A.
(c) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 22 minutes to 24 minutes is not more than 50 ppm to the absorption peak area of the bisphenol A.
(d) A high-quality bisphenol A wherein the sum total of the contents of the 1-naphthol derivatives represented by following formula (1) is not more than 200 ppm to the bisphenol A; 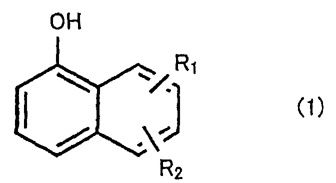 wherein R₁ and R₂ each represents a methyl group, an ethyl group, an n-propyl group, an isopropyl group, or an isopropenyl group each bonded to the 2 to 8 position of the 1-naphthol.
(e) A high-quality bisphenol A wherein the content of 9,9'-dimethylxanthene is not more than 2 ppm.
(f) A high-quality bisphenol A wherein the content of 2-(4-hydroxyphenyl)-2,4,4-trimethylchroman is not more than 50 ppm.
(g) A high-quality bisphenol A wherein the content of 2,2,4-trimethylchromene is not more than 5 ppm.

In the case that the high-quality bisphenol A of the invention is (a) described above, the content of sodium is preferably not more than 50 ppb and the content of each element of the group A consisting of iron, nickel, chromium, manganese, lead, and molybdenum is preferably not more than 20 ppb, and more preferably not more than 10 ppb. When the content of sodium contained in bisphenol A is not more than 80 ppb and the content of each element of the group A is not more than 50 ppb, in the case of preparing an aromatic polycarbonate using the bisphenol A, coloring of the aromatic polycarbonate obtained is improved and further coloring at molding the aromatic polycarbonate obtained is also improved.

Also, in the case that the high-quality bisphenol A of the invention is (a) described above, in addition to each element of above-described group A, when the content of each element of group B consisting of copper, zinc, aluminum, and indium or the content of silicon contained in the bisphenol A is not more than 20 ppb, preferably not more than 10 ppb, and more preferably not more than 5 ppb, and when the content of titanium contained in the bisphenol A is not more than 1 ppb, coloring of the aromatic polycarbonate obtained from the bisphenol A can be more effectively improved.

In addition, the content of each metal in the invention is measured by a flameless atomic absorption spectrometry (electric heating atomic absorption spectrometry). It is preferred that as a vessel for sampling from the preparation equipment of bisphenol A for measuring the content of each metal, a vessel sufficiently washed so that each metal is not intermixed from the vessel is used and after sampling, the vessel is tightly sealed and is not opened before carrying out the analysis. By carrying out the operation from the pretreatment to the measurement in a clean environment, contamination by each meal in the atmosphere can be prevented.

When the high-quality bisphenol A of the invention is (b) described above, by the high performance liquid chromatographic analysis described below, the sum total of the absorption peak areas of the compounds eluted between the time of from 15.5 minutes to 24 minutes (hereinafter, the compounds are referred to as compound group A) is not more than 2 × 10⁻³, and preferably not more than 1 × 10⁻³ to the absorption peak area of the bisphenol A. When the sum total of the absorption peak areas of the compound group A is not more than 2 × 10⁻³ to the absorption peak area of the bisphenol A, coloring at the preparation of the aromatic polycarbonate is improved and further coloring at molding the aromatic polycarbonate obtained is also improved.

When the high-quality bisphenol A of the invention is (c) described above, by the high performance liquid chromatographic analysis described below, the sum total of the absorption peak areas of the compounds eluted between the time of from 22 minutes to 24 minutes (hereinafter, the compounds are referred to as compound group B) is not more than 50 ppm, and preferably not more than 30 ppm to the absorption peak area of the bisphenol A. When the sum total of the absorption peak areas of the compound group B is not more than 50 ppm of the absorption peak area of the bisphenol A, coloring at the preparation of the aromatic polycarbonate is improved and further coloring at molding the aromatic polycarbonate obtained is also improved.

Also, it is preferred that in the compound group B, the sum total of the absorption peak areas of the compounds having molecular weights of from 307 to 309 is not more than 20 ppm, and more preferably not more than 10 ppm of the absorption peak area of the bisphenol A. In the compound group B, the compounds having molecular weights of from 307 to 309 give particularly large influences on the hue of the aromatic polycarbonate and when these compounds are not more than 20 ppm, coloring at the preparation of the aromatic polycarbonate is improved and further coloring at molding the aromatic polycarbonate obtained is also improved.

In addition, the details of the analytic method by the high performance liquid chromatography in the invention are as follows.

There is no particular restriction on the high performance liquid chromatographic apparatus and a generally commercially available apparatus may be used but the apparatus equipped with a degassing unit capable of sufficiently carrying out degassing of the eluent is used, and as a liquid sending pump, a double plunger type low pulsating pump capable of carrying out the gradient operation is used. Also, for heat retaining the analytical column at a constant temperature, the analytical column is installed in a column oven capable of temperature control in the range of within ± 0.1°C. As the ultraviolet detector, a ultraviolet detector capable of measuring at the accuracy of the wavelength reproducibility ± 0.1 nm is used, when the high-quality bisphenol A is (b), (c), or (d) described above, the measurement is carried out at a wavelength of 254 nm, when the high-quality bisphenol A is (e) or (g), the measurement is carried out at a wavelength of 200 nm, and when the high-quality bisphenol A is (f), the measurement is carried out at a wavelength of 280 nm. As an embodiment of such a high performance liquid chromatographic apparatus, an LC-10A type high performance liquid chromatographic apparatus manufactured by SHIMADZU CORPORATION can be suitably used.

As the analytical column, a stainless steel-made column having an inner diameter of 4.6 mm and a length of 250 mm packed with a high-pure spherical silica gel having a fine pore of 100 angstroms bonded with an octadecyl group in an amount of 15% by weight of the silica gel, such as, for example, Inertsil ODS-3 type adsorbent manufactured by GL Science Corporation as the absorbent is used. The analytical column is retained at a temperature range of 40°C ± 0.1°C and then used for the analysis.

As the eluents, an aqueous 0.1% phosphoric acid solution is used as an eluent A, acetonitrile is used as an eluent B, a measurement is carried out such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then continuously increasing the rate of the eluent B such that the ratio of the eluent A : the eluent B becomes 0 : 1 until 55 minutes after the initiation of the measurement is carried out.

As the aqueous 0.1% phosphoric acid solution as the eluent A, phosphoric acid of higher than the JIS guaranteed reagent is diluted to 0.1 ± 0.0001 (%) with commercially available distilled water for a high performance liquid chromatography and is used. As acetonitrile as the eluent B, commercially available acetonitrile for high performance liquid chromatography is used.

When the high-quality bisphenol A of the invention is (d) described above, the sum total of the contents of the 1-naphthol derivatives shown by the above-described formula (1) contained in the bisphenol A is not more than 200 ppm, and more preferably not more than 100 ppm. When the contents of the 1-naphthol derivatives contained in the bisphenol A are not more than 200 ppm, coloring at the preparation of the aromatic polycarbonate is improved and further coloring at molding the aromatic polycarbonate obtained is also improved.

Practical examples of the 1-naphthol derivative shown by the formula (1) include 5,7-dimethyl-l-naphthol, 6,7-dimethyl-1-naphthol, 5,8-dimethyl-1-naphthol, 6,8-dimethyl-1-naphthol, 5-ethyl-7-methyl-1-naphthol, 5-methyl-7-ethyl-1-naphthol, 6-ethyl-7-methyl-1-naphthol, 6-methyl-7-ethyl-1-naphthol, 5,7-diethyl-1-naphthol, 6,7-diethyl-1-naphthol, 5-n-propyl-7-methyl-1-naphthol, 6-n-propyl-7-methyl-1-naphthol, 5-isopropyl-7-methyl-1-naphthol, 6-isopropyl-7-methyl-1-naphthol, 5-isopropenyl-7-methyl-1-naphthol, and 6-isopropenyl-7-methyl-1-naphthol.

Also, it is preferred that the sum total of the contents of the 1-naphthol derivatives shown in the formula (1) contained in the bisphenol A is not more than 200 ppm, and also when the bisphenol is analyzed by the above-described high performance liquid chromatographic analysis, the sum total of the absorption peak areas of the compounds (Compound group B) eluted between the time of from 22 minutes to 24 minutes is not more than 50 ppm, and more preferably not more than 30 ppm to the area of the absorption peak of the bisphenol A, because in this case, coloring of the aromatic polycarbonate obtained from the bisphenol A becomes less.

When the high-quality bisphenol A of the invention is (e) described above, the content of 9,9'-dimethylxanthene contained in the bisphenol A is not more than 2 ppm, and preferably not more than 1 ppm. When the content of 9,9'-dimethylxanthene contained in the bisphenol A is not more than 2 ppm, coloring at the preparation of the aromatic polycarbonate is improved and further coloring at molding the aromatic polycarbonate obtained is also improved.

When the high-quality bisphenol A of the invention is (f) described above, the content of 2-(4-hydroxyphenyl)-2,4,4-trimethylchroman contained in the bisphenol A is not more than 50 ppm, preferably not more than 30 ppm, and more preferably not more than 20 ppm. When the content of 2-(4-hydroxyphenyl)-2,4,4-trimethylchroman contained in the bisphenol A is not more than 50 ppm, coloring at the preparation of the aromatic polycarbonate is improved and further coloring at molding the aromatic polycarbonate obtained is also improved.

Also, it is preferred that the content of 2-(4-hydroxyphenyl)-2,4,4-trimethylchroman contained in the bisphenol A is not more than 50 ppm as well as the content of sodium is not more than 80 ppb, and the content of each element of group A consisting of iron, nickel, chromium, manganese, lead, and molybdenum is not more than 50 ppb, preferably not more than 20 ppb, and more preferably not more than 10 ppb because in this case, coloring of the aromatic polycarbonate prepared from the bisphenol A becomes more less.

When the high-quality bisphenol A of the invention is (g) described above, the content of 2,2,4-trimethylchromene contained in the bisphenol A is not more than 5 ppm, and preferably not more than 2 ppm. When the content of 2,2,4-trimethylchromene contained in the bisphenol A is not more than 5 ppm, coloring at the preparation of the aromatic polycarbonate is improved and further coloring at molding the aromatic polycarbonate obtained is also improved.

Also, in the case that the high-quality bisphenol A of the invention is (b), (c), (d), (e), or (g), when the content of iron contained in the bisphenol A is not more than 50 ppb, preferably not more than 30 ppb, and more preferably not more than 10 ppb, the deterioration of the hue of the aromatic polycarbonate obtained from the bisphenol A can be prevented.

As a process for preparing the high-quality bisphenol A of the invention as (a) described above, there is a process for removing the catalyst from the reaction mixture and after, if necessary, removing unreacted acetone and water formed in the reaction, contacting the reaction mixture with an absorbent. Examples of the absorbent used include active clay, diatomaceous earth, montmorillonite, molecular sieve, clay minerals, active carbon, graphite, hydrotalcite, chelate resins, and cation-exchange resins.

Also, after, if necessary, removing unreacted acetone and water formed in the reaction from the reaction mixture, the reaction mixture is cooled, and by contacting at least a part of the mother liquor after separating the adducts therefrom with the above-described absorbent, the contents of sodium and each element of group A can be reduced.

Or, the crystallization operation for obtaining the adducts by cooling the reaction mixture is usually once or twice in the preparation process of the bisphenol A but by increasing the number of times of the crystallization operation, the contents of sodium and each element of group A in the bisphenol A finally obtained can be more reduced. Also, the plural operations described above may be combined at use.

By carrying out each operation described above, the contents of copper, zinc, aluminum, and indium as the group B elements, and silicon and titanium can be simultaneously reduced.

As a process for preparing the bisphenol A, wherein the contents of organic compounds as impurities are reduced, as the high-quality bisphenol A of the invention of (b) to (g), there is a process wherein in the preparation process of bisphenol A, the number of times of the crystallization operation, which is usually carried out once or twice, is more increased once or twice. There is no particular restriction of the method of the crystallization, but it is preferred that in the case of separating a mother liquor from the adducts precipitated by the crystallization, the adducts are washed with phenol.

Because an aromatic polycarbonate is excellent in the impact resistance and the transparency, the polycarbonate is largely used for optical uses, particularly, as optical disk substrates, and recently, by the increase of the recording density and the recording capacity of the optical disks, the demand for the reliability of recording and regeneration of optical disks has been increased. The influence of the hue of the aromatic polycarbonate as the raw material of optical disks on the reliability of recording and regeneration of optical disks is large and it is desirable that the aromatic polycarbonate is as colorless and transparent as possible.

In general, as the evaluation of the hue of a polymer, a b value showing a yellowness is used, and a smaller b value shows that coloring is less. The preferred b value as the aromatic polycarbonate for optical use is not larger than 1.0, more preferably not larger than 0.5, and far more preferably not larger than 0.3. By using the high-quality polycarbonate of the invention, the aromatic polycarbonate showing the hue of the above-describe range can be prepared.

The high-quality polycarbonate of the invention gives less coloring at the preparation or molding of the aromatic polycarbonate and is particularly excellent as the raw material of the aromatic polycarbonate required to be colorless and transparent, in the case of optical uses, etc. Also, as the preparation process of an aromatic polycarbonate, there are a phosgene process, a transesterification process, etc., and the bisphenol A of the invention can be used for any one of these processes. Particularly, in the case of preparing an aromatic polycarbonate by the transesterification process which is usually liable to cause coloring, in the case of using the bisphenol A of the invention, the occurrence of coloring is restrained and thus the bisphenol of the invention can be suitable used.

### EXAMPLES

Then, the invention is explained in more detail by the following examples. In addition, each analysis was carried out as follows.
(1) Preparation of test liquid for analyzing each element:
   In a clean room of class 1,000, 5 g of bisphenol A accurately weighed was placed on a platinum plate, after sublimating organic substances by heating using an electric heater, the rest was cooled, 0.5 ml of sulfuric acid was added thereto, and after carbonizing the rest by heating again, the carbonized rest was incinerated by a gas burner. After allowing to cool, 2 ml of 17% hydrochloric acid was added, followed by heating to dissolve the residue, and after adding water thereto and heating, a test liquid of a constant volume of 10 ml was prepared. In addition, as the reagents used for the pre-treatments, commercially available reagents for electronic industry were used, and the instruments were subjected to an immersion treatment in diluted nitric acid, etc., for a sufficient time and thereafter were washed with ultrahigh purity water before use.
(2) Analysis of each element:
   The test liquid obtained in (1) described above was analyzed by a SIMAA 6000 type flameless atomic absorption spectrometric analytical apparatus manufactured by PERKIN ELMER CORPORATION.
(3) Determination of each organic compound:
   In 3 ml of acetonitrile was dissolved 2 g of bisphenol A, using an LC-10A type liquid chromatography manufactured by SHIMADZU CORPORATION, according to the above-described method, 2,2,4-trimethylchromene and 9,9'-dimethylxanthene were determined by a ultraviolet detector of a wavelength of 200 nm, 2-(4-hydroxyphenyl)-2,4,4-trimethylchroman was determined by a ultraviolet detector of a wavelength of 280 nm, and other organic compounds were determined by a ultraviolet detector of a wavelength of 254 nm.
(4) Measurement of the b value of aromatic polycarbonate:
   The b value of the press sheet of 2 mm in thickness was measured by a transmission method using an HSC-5N type color meter manufactured by Suga Shikenki K.K.

### Example 1

Using hydrogen chloride as a catalyst, by reacting acetone and phenol at a mol ratio of 1 : 6 at a reaction temperature of 60°C, a reaction mixture was obtained. The reaction mixture was distilled to remove hydrogen chloride and further was brought into contact with a sulfonic acid-base cation-exchange resin. The reaction mixture was cooled to 45°C to precipitate adducts and the adducts formed were separated from the mother liquor. The adducts were heated to 180°C at 40 Torr to remove phenol until the phenol concentration ir bisphenol A became 3% and further phenol was removed therefron by steam stripping to obtain a bisphenol A. The content of each element in the bisphenol A are shown in Table 1. Using tetramethylammonium hydroxide as a catalyst, 102.6 g of the bisphenol A was reacted with 105.9 g of diphenyl carbonate with stirring by reducing the pressure to 15 mmHg and increasing the temperature to 240°C. Then, the reaction of the reaction product obtained was carried out in a thin-film type reactor at a pressure of 4 mmHg and a temperature of 260°C to obtain a prepolymer. The prepolymer was further polymerized at a temperature of 280°C and a pressure of 0.5 mmHg to obtain a polycarbonate. The intrinsic viscosity of the polycarbonate obtained was 0.35 and the b value thereof was 0.1.

### Example 2

Using hydrogen chloride as a catalyst, by reacting acetone and phenol at a mol ratio of 1 : 6 at a reaction temperature of 60°C, a reaction mixture was obtained. After distilling the reaction mixture to remove hydrogen chloride, the reaction mixture was cooled to 45°C to precipitate adducts and adducts were separated from the mother liquor. After purging 10% of the total amount of the mother liquor thus separated from the system, the mother liquor was brought into contact with a sulfonic acid type cation-exchange resin and recycled to the reactor. The state was continued until the concentrations of the organic impurities in the system became constant, and when the concentrations of the organic impurities became constant, the adducts obtained were dissolved in phenol and a recrystallization was carried out at 45°C to obtain a purified adduct. The purified adduct was heated to 180°C at a pressure of 40 Torr to remove phenol until the phenol concentration on bisphenol A became 3% and further, phenol was removed by steam stripping to obtain a bisphenol A. The content of each element in the bisphenol A is shown in Table 1. When the polymerization was carried out as in Example 1 using the bisphenol as a raw material, a polycarbonate was obtained. The intrinsic viscosity of the polycarbonate obtained was 0.35 and the b value thereof was 0.2.

### Comparative Example 1

By following the same procedure as Example 1 except that the cation-exchange resin treatment of the reaction mixture after removing hydrogen chloride was not carried out, a bisphenol A was obtained. The content of each element in the bisphenol is shown in Table 1. When the polymerization was carried out as in Example 1 using the bisphenol A as the raw material, a polycarbonate was obtained. The intrinsic viscosity of the polycarbonate obtained was 0.34 and the b value thereof was 2.5.

### Comparative Example 2

To the bisphenol A obtained in Example 1 was added an aqueous 0.01% sodium hydroxide solution such that the content of sodium to the bisphenol A became 100 ppb. After melting the bisphenol A and sufficiently mixing, diphenyl carbonate and tetramethylammonium hydroxide were added to the mixture and the polymerization was carried out as in Example 1. The intrinsic viscosity of the polycarbonate obtained was 0.33 and the b value thereof was 1.1.

### Comparative Examples 3 to 7

By following the same procedure as Comparative Example 2 except that an aqueous 0.01% iron chloride solution, an aqueous 0.01% nickel chloride solution, an aqueous 0.01% chromium chloride solution, or an aqueous manganese chloride solution was added in place of an aqueous 0.01% sodium hydroxide solution such that the content of each element of the group A in the bisphenol A became 70 ppb, each polycarbonate was obtained. The intrinsic viscosities and the b values of the polycarbonate obtained are shown in Table 1.

### Example 3

By following the same procedure as Comparative Example 2 except that the content of sodium to the bisphenol A was adjusted to become 40 ppb, a polycarbonate was obtained. The intrinsic viscosity of the polycarbonate obtained was 0.34 and the b value thereof was 0.6.

### Examples 4 to 13

By following the same procedures as Comparative Examples 3 to 6 except that the addition amounts of the aqueous solutions of the chlorides of the elements of group A were controlled such that each of the contents of the elements of the group A in the bisphenol A became 30 ppb and 15 ppb, polycarbonates were obtained. The intrinsic viscosities and the b values of the polycarbonates obtained are shown in Table 1.

### Examples 14 to 17

By following the same procedure as Comparative Example 2 except that an aqueous 0.005% copper chloride solution, an aqueous 0.005% zinc chloride solution, an aqueous 0.005% aluminum chloride solution, or an aqueous 0.005% indium chloride solution was added in place of the aqueous 0.01% sodium hydroxide solution such that the content of each of the elements of the group B became 25 ppb, polycarbonates were obtained. The intrinsic viscosities and the b values of the polycarbonates obtained are shown in Table 1.

### Example 18

By following the same procedure as Comparative Example 2 except that an aqueous 0.001% titanium chloride solution was added in place of the aqueous 0.01 sodium hydroxide solution such that the content of titanium in the bisphenol A became 5 ppb, a polycarbonate was obtained. The intrinsic viscosity and the b value of the polycarbonate obtained are shown in Table 1.

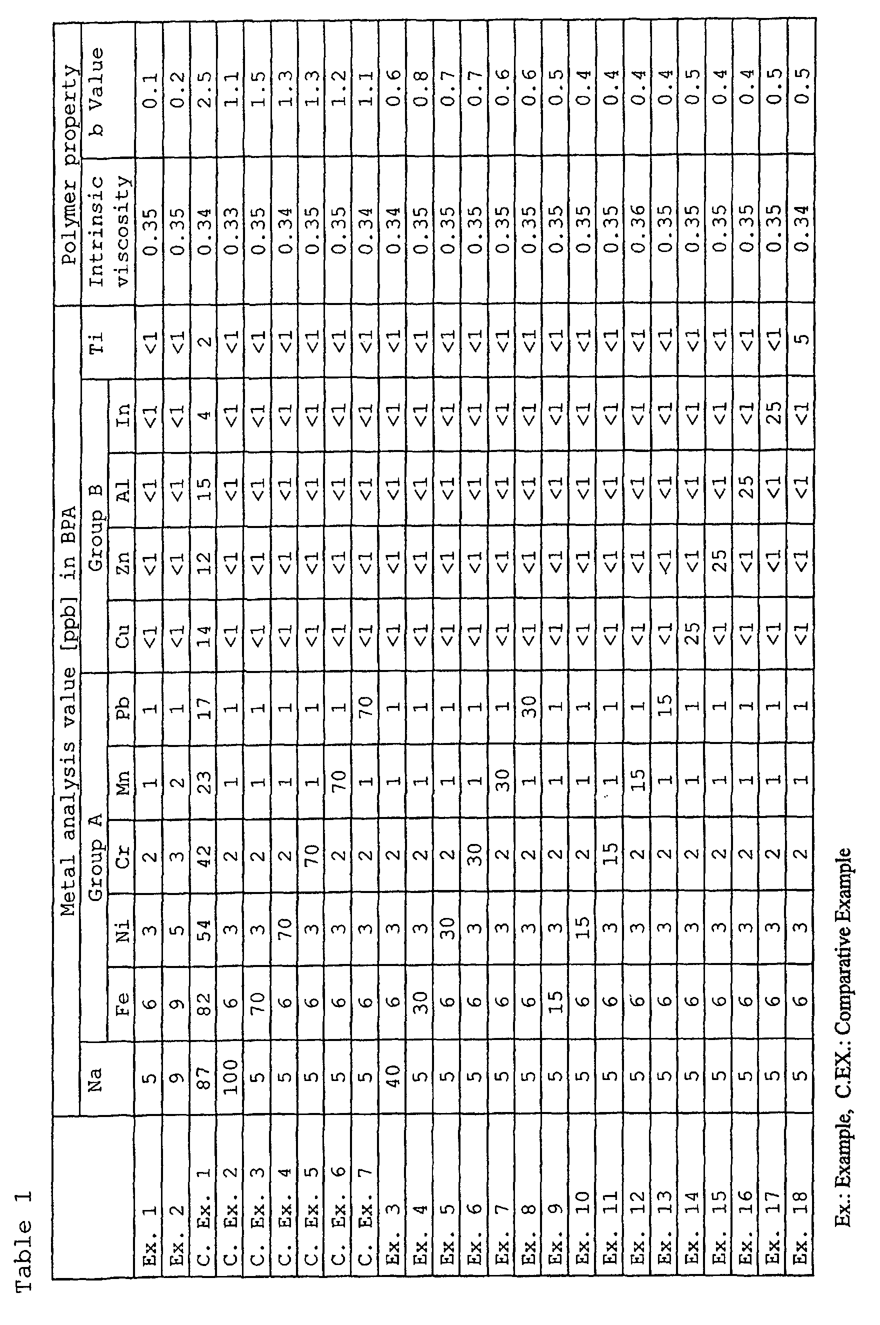

### Example 19

Using a reactor of which the inside surface was lined with glass and using hydrogen chloride as a catalyst, acetone was reacted with phenol at a mol ratio of 1 : 6 at a reaction temperature of 60°C, a reaction mixture was obtained. The reaction mixture was distilled to remove hydrogen chloride and further the reaction mixture was brought into contact with a sulfonic acid-base cation-exchange resin. The reaction mixture was cooled to 45°C to precipitate adducts and the adducts obtained were separated from the mother liquor. The adducts were heated to 180°C at a pressure of 40 Torr to remove phenol until the phenol concentration in bisphenol A became 3% and further phenol was removed by steam stripping to obtain a bisphenol A. The content of each element in the bisphenol A is shown in Table 2. Using tetramethylammonium hydroxide as a catalyst, 102.6 g of the bisphenol A was reacted with 105.9 g of diphenyl carbonate with stirring by reducing the pressure to 15 mmHg and increasing the temperature to 240°C. Then, the reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain a polycarbonate. The intrinsic viscosity of the polycarbonate obtained was 0.35 and the b value thereof was 0.1.

### Example 20

Using a reactor of which the inside surface was lined with glass and using hydrogen chloride as a catalyst, acetone was reacted with phenol at a mol ratio of 1 : 6 at a reaction temperature of 60°C, a reaction mixture was obtained. After distilling the reaction mixture to remove hydrogen chloride, the reaction mixture was cooled to 45°C to precipitate adducts and the adducts obtained were separated from the mother liquor. After purging 10% of the total amount of the mother liquor thus separated from the system, the mother liquor was brought into contact with a sulfonic acid type cation-exchange resin and recycled to the reactor. The state was continued until the concentrations of the organic impurities in the system became constant, and when the concentrations of the organic impurities became constant, the adducts obtained were dissolved in phenol and a recrystallization was carried out at 45°C to obtain a purified adduct. The purified adduct was heated to 180°C at a pressure of 40 Torr to remove phenol until the phenol concentration in the bisphenol A became 3%, and further phenol was removed by steam stripping to obtained a bisphenol A. The content of each element in the bisphenol A is shown in Table 2. When the polymerization was carried out as in Example 19 using the bisphenol A as the raw material, a polycarbonate was obtained. The intrinsic viscosity of the polycarbonate obtained was 0.35 and the b value thereof was 0.2.

### Comparative Example 8

By following the same procedure as Example 19 except that the cation-exchange resin treatment of the reaction mixture after removing hydrogen chloride was not carried out, a bisphenol A was obtained. The content of each element in the bisphenol A is shown in Table 2. When the polymerization was carried out as in Example 19 using the bisphenol A as the raw material, a polycarbonate was obtained. The intrinsic viscosity of the polycarbonate obtained was 0.34 and the b value thereof was 2.5.

### Comparative Example 9

An aqueous 0.01% sodium hydroxide solution was added to the bisphenol A obtained in Example 19 such that the content of sodium in the bisphenol A became 100 ppb. After melting the bisphenol A and sufficiently mixing, diphenyl carbonate and tetramethylammonium hydroxide were added thereto and the polymerization was carried out as in Example 19. The intrinsic viscosity of the polycarbonate obtained was 0.33 and the b value thereof was 1.1.

### Comparative Examples 10 to 14

By following the same procedure as Comparative Example 9 except that an aqueous 0.01% iron chloride solution, an aqueous 0.01% nickel chloride solution, an aqueous 0.01% chromium chloride solution, an aqueous manganese chloride solution, or an aqueous sodium molybdate was added in place of the aqueous 0.01% sodium hydroxide solution such that the content of each element of the group A in the bisphenol A became 70 ppb, polycarbonates were obtained. The intrinsic viscosities and the b values of the polycarbonates obtained are shown in Table 2.

### Example 21

By following the same procedure as Comparative Example 9 except that the content of sodium in the bisphenol A was controlled to become 40 ppb, a polycarbonate was obtained. The intrinsic viscosity of the polycarbonate obtained was 0.34 and the b value thereof was 0.6.

### Examples 22 to 31

By following the same procedures as Comparative Examples 10 to 13 except that the addition amount of each of the aqueous solutions of the elements of the group A was controlled such that the contents of the elements of the group A became 30 ppb and 15 ppb, polycarbonates were obtained. The intrinsic viscosities and the b values of the polycarbonates obtained are shown in Table 2.

### Example 32

By following the same procedure as Comparative Example 9 except that an aqueous 0.005% sodium metasilicate was added in place of the aqueous 0.01% sodium hydroxide solution such that the content became 25 ppb, a polycarbonate was obtained. The intrinsic viscosity and the b value of the polycarbonate obtained are shown in Table 2.

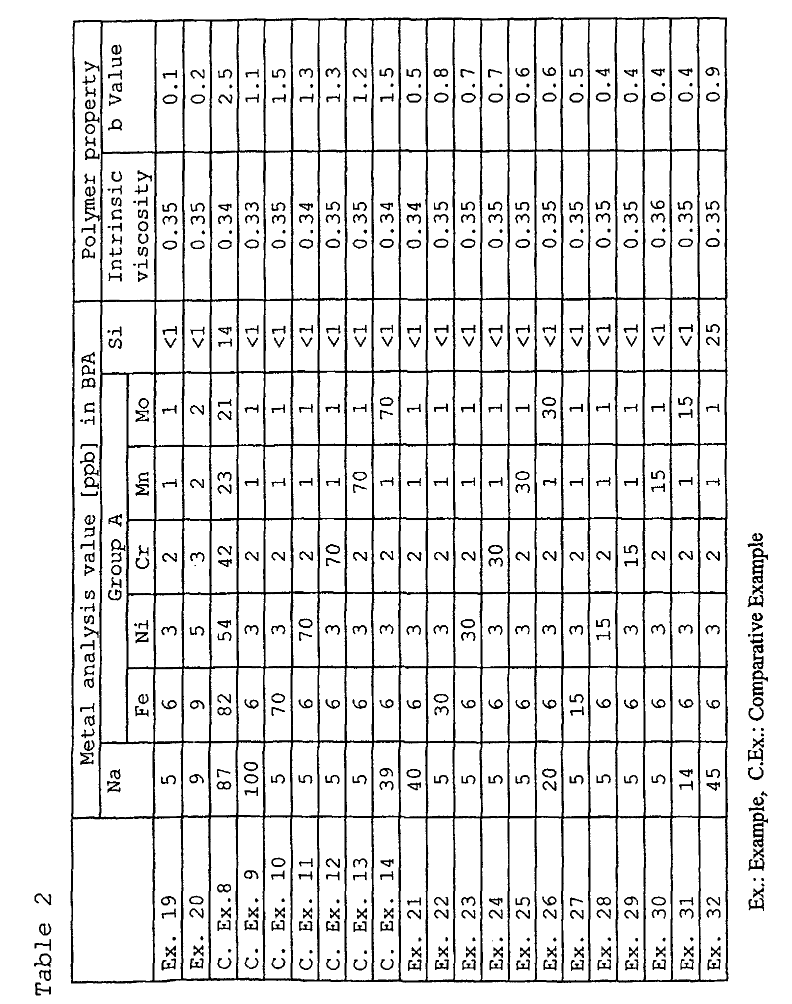

### Example 33

Using a sulfonic acid-base ion-exchange resin of a crosslinking degree of 4%, of which 15% of the whole sulfonic acid groups was modified by cysteamine as a catalyst, acetone was reacted with phenol at mol ratio of 1 : 11 at a reaction temperature of 75°C to obtain a reaction mixture. The reaction mixture was cooled to 45°C to precipitate adducts (the 1st crystallization) and the adducts obtained were separated from the mother liquor. After purging 10% of the total amount of the mother liquor separated from the system, water was removed from the mother liquor and the mother liquor was recycled to the reactor. The state was continued until the concentrations of the organic impurities in the system became constant, when the concentrations of the organic impurities became constant, the adducts obtained were dissolved in phenol and the recrystallization was carried out at 45°C (the 2nd crystallization) to obtain adducts. The adducts obtained were further dissolved in phenol to carry out the 3rd crystallization, the adducts obtained were heated to 180°C at a pressure of 40 Torr to remove phenol until the phenol concentration in the bisphenol A became 3% and further phenol was removed by steam stripping to obtain a bisphenol A. The bisphenol A obtained was analyzed by a liquid chromatography. Also, iron in the bisphenol A obtained was analyzed.

The reaction of 102.6 g of the bisphenol A and 105.9 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain an aromatic polycarbonate. As the result thereof, the intrinsic viscosity [η] of the aromatic polycarbonate obtained was 0.35 and the b value showing the hue of the polymer was 0. The results are shown in Table 3.

### Comparative Example 15

By following the same procedure as Example 33 except that the number of the crystallization was changed to twice, a bisphenol A was obtained. The bisphenol A obtained was analyzed by the liquid chromatography. Also, the content of iron analyzed was 25 ppb. Furthermore, when an aromatic polycarbonate was obtained by polymerization using the bisphenol A by the same manner as in Example 33, the intrinsic viscosity of the aromatic polycarbonate obtained was 0.34 and the b value showing the hue of polymer was 1.5. The results are shown in Table 3.

**Table 3**

| | Organic Impurities (weight ppm) | | | Ratio of of peak areas of compound group A to peak of | Fe (ppb) | Polymer b value |
|---|---|---|---|---|---|---|
| | Phenol | OP'-BPA | Chromans | | | |
| Ex.1 | 25 | 57 | 28 | 0.4 × 10⁻³ | 4 | 0 |
| C.Ex.15 | 23 | 326 | 135 | 2.6 × 10⁻³ | 25 | 1.5 |
| OP'-BPA: 2-(2-Hydroxyphenyl)-2-(4-hydroxyphenyl)propane Ex.: Example C.Ex.: Comparative Example | | | | | | |

### Examples 34 and 35

When each aromatic polycarbonate was obtained by a polymerization by the same manner as Example 33 except that to the bisphenol A obtained in Example 33 were added the compounds of the compound group A obtained by preparative chromatography from the reaction mixture obtained in Example 33 such that the sum total of the absorption peak areas by the liquid chromatography of the compound group A became 0.9 x 10⁻³ and 1.5 × 10⁻³ respectively to the absorption peak area of the bisphenol A, the intrinsic viscosities of the aromatic polycarbonates obtained were 0.35 and 0.35 respectively and the b values were 0.2 and 0.6 respectively.

### Comparative Example 16

When an aromatic polycarbonate was prepared by carrying out the polymerization by the following the same procedure as Example 34 except that the compounds of the compound group A were added such that the sum total of the absorption peak areas of the compounds of the compound group A by the liquid chromatography became 2.5 x 10⁻³ to the absorption peak area of the bisphenol A, the intrinsic viscosity of the aromatic polycarbonate obtained was 0.34 and the b value thereof was 1.1.

### Example 36

Using a sulfonic acid-base ion-exchange resin of a crosslinking degree of 4%, of which 17% of the whole sulfonic acid groups was modified by cysteamine as a catalyst, acetone was reacted with phenol at mol ratio of 1 : 11 at a reaction temperature of 80°C to obtain a reaction mixture. The reaction mixture was cooled to 45°C to precipitate adducts (the 1st crystallization) and the adducts obtained were separated from the mother liquor. After purging 10% of the total amount of the mother liquor separated from the system, water was removed from the mother liquor and the mother liquor was recycled to the reactor. The state was continued until the concentrations of the organic impurities in the system became constant, when the concentrations of the organic impurities became constant, the adducts obtained were dissolved in phenol and the recrystallization was carried out at 45°C (the 2nd crystallization) to obtain adducts. The adducts obtained were further dissolved in phenol to carry out the 3rd crystallization, the adducts obtained were heated to 180°C at a pressure of 40 Torr to remove phenol until the phenol concentration in the bisphenol A became 3% and further phenol was removed by steam stripping to obtain a bisphenol A. When the bisphenol A obtained was analyzed by the liquid chromatography, the sum total the absorption peak areas of the compound group B was 15 ppm to the absorption peak area of the bisphenol A. In addition, in the compound group B, the absorption peak area of the compound having a molecular weight of 308 detected was 9 ppm to the absorption peak area of bisphenol A. Also, the iron in the bisphenol A obtained was analyzed.

The reaction of 102.6 g of the bisphenol A and 105.9 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain an aromatic polycarbonate. As the result thereof, the intrinsic viscosity [η] of the aromatic polycarbonate obtained was 0.35 and the b value showing the hue of the polymer was 0.1. The results are shown in Table 4.

### Comparative Example 17

By following the same procedure as Example 36 except that the number of the crystallization was changed to twice, a bisphenol A was obtained. When the bisphenol A obtained was analyzed by the liquid chromatography, the sum total of the absorption peak areas of the compound group B was 88 ppm to the absorption peak area of the bisphenol A. In addition, in the compound group B, the absorption peak area of the compound having a molecular weight of 308 detected was 53 ppm to the absorption peak area of bisphenol A.

Also, the content of iron analyzed was 25 ppb. Furthermore, when using the bisphenol A, a polycarbonate was obtained by polymerizing as in Example 36, the intrinsic viscosity of the polycarbonate obtained was 0.34 the b value thereof showing the hue of the polymer was 1.7. The results are shown in Table 4.

**Table 4**

| | Organic Impurity (weight ppm) | | | Ratio of area to peak area of BPA | | | |
|---|---|---|---|---|---|---|---|
| | Phenol | OP'-BPA | Chromans | Sum total of compound group B | Compound of molecula r weight 308 | Fe (ppb) | Polymer b value |
| | 32 | 57 | 31 | 15 ppm | 9 ppm | 4 | 0.1 |
| C.Ex. 17 | 28 | 342 | 143 | 88 ppm | 53 ppm | 25 | 1.7 |
| OP'-BPA: 2-(2-Hydroxyphenyl)-2-(4-hydroxyphenyl)propane Ex.: Example C.Ex.: Comparative Example | | | | | | | |

### Examples 37 and 38

When each aromatic polycarbonate was obtained by a polymerization by the same manner as Example 33 except that to the bisphenol A obtained in Example 36 were added the compounds of the compound group A obtained by preparative chromatography from the reaction mixture obtained in Example 36 such that the sum total of the compound group B became 25 ppm, and 40 ppm respectively to the bisphenol A, the intrinsic viscosities of the aromatic polycarbonates obtained were 0.35 and 0.35 respectively and the b values were 0.2 and 0.6 respectively.

### Comparative Example 18

By following the same procedure as Example 37 except that the compounds of the compound group B were added to the bisphenol A such that the sum total of the compound group B became 70 ppm to the bisphenol A, an aromatic polycarbonate was obtained by polymerization, the intrinsic viscosity of the polycarbonate obtained was 0.34 and the b value thereof was 1.2.4

### Example 39

Using a sulfonic acid-base ion-exchange resin of which 15% of the whole sulfonic acid groups was modified by cysteamine as a catalyst, acetone was reacted with phenol at mol ratio of 1 : 12 at a reaction temperature of 75°C to obtain a reaction mixture. The reaction mixture was cooled to 45°C to precipitate adducts (the 1st crystallization) and the adducts obtained were separated from the mother liquor. After purging 10% of the total amount of the mother liquor separated from the system, water was removed from the mother liquor and the mother liquor was recycled to the reactor. The state was continued until the concentrations of the organic impurities in the system became constant, when the concentrations of the organic impurities became constant, the adducts obtained were dissolved in phenol and the recrystallization was carried out at 45°C (the 2nd crystallization) to obtain adducts. The adducts obtained were further dissolved in phenol to carry out the 3rd crystallization, the adducts obtained were heated to 180°C at a pressure of 40 Torr to remove phenol until the phenol concentration in the bisphenol A became 3% and further phenol was removed by steam stripping to obtain a bisphenol A. As the result of analyzing the bisphenol A obtained by the liquid chromatography, 5,7-dimethyl-1-naphthol and 5,7-dimethyl-1-naphthol were detected as 1-naphthol derivatives and the sum total of them was 46 ppm. Also, when the analysis of the compound group B and iron was carried out, they were 11 ppm and 2 ppb respectively.

The reaction of 102.6 g of the bisphenol A and 105.9 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain a polycarbonate. As the result thereof, the intrinsic viscosity [η] of the polycarbonate obtained was 0.35 and the b value showing the hue of the polymer was 0. The results are shown in Table 5.

### Comparative Example 19

By following the same procedure as Example 39 except that the number of the crystallization was changed to twice, a bisphenol A was obtained.

As the result of analyzing the bisphenol A obtained by the liquid chromatography as in Example 39, the sum total of the 1-naphthol derivatives was 294 ppm. Also, when the analysis of the compound group B and iron was carried out, they were 72 ppm and 22 ppb respectively. Furthermore, when using the bisphenol A, a polycarbonate was obtained by the polymerization as in Example 39, the intrinsic viscosity of the polycarbonate obtained was 0.36 and the b value showing the hue of the polymer was 1.5. The results are shown in Table 5.

### Examples 40 and 41

When each aromatic polycarbonate was obtained by the polymerization by the same manner as Example 39 except that to the bisphenol A obtained in Example 39 was added 7-dimethyl-1-naphthol obtained by preparative chromatography from the reaction mixture obtained in Example 39 such that the sum total amount of the 1-naphthol derivative became 80 ppm and 150 ppm respective to the bisphenol A, the intrinsic viscosities of the aromatic polycarbonates obtained were 0.35 and 0.36 respectively and the b values were 0.2 and 0.7 respectively.

### Comparative Example 20

By following the same procedure as Example 40 except that the addition amount of 5,7-dimethyl-1-naphthol was changed such that the sun total amount of the 1-naphthol derivative became 250 ppm to the bisphenol A, an aromatic polycarbonate was obtained by the polymerization. The intrinsic viscosity of the polycarbonate obtained was 0.35 and the b value thereof was 1.1.

### Example 42

By following the same procedure as Example 40 except that 6,7-dimethyl-1-naphthol was added in place of 5,7-dimethyl-1-naphthol such that the sum total amount of the 1-naphthol derivative became 80 ppm to the bisphenol A, an aromatic polycarbonate was obtained by the polymerization. The intrinsic viscosity of the polycarbonate obtained was 0.35 and the b value thereof was 0.3.

### Comparative Example 21

By following the same procedure as Example 40 except that the addition amount of 6,7-dimethyl-1-naphthol was changed such that the sum total amount of the 1-naphthol derivative became 250 ppm to the bisphenol A, an aromatic polycarbonate was obtained by the polymerization. The intrinsic viscosity of the polycarbonate obtained was 0.35 and the b value thereof was 1.2

**Table 5**

| | Organic Impurity (weight ppm) | | | Ratio of sum total of 1-naphtho 1 to BPA | Ratio of peak areas of compound group B to peak area of BPA | Fe (ppb) | Polymer b value |
|---|---|---|---|---|---|---|---|
| | Phenol | OP'-BPA | Chromans | | | | |
| Ex 39 | 25 | 48 | 25 | 46 ppm | 11 ppm | 2 | 0 |
| C. Ex 19 | 23 | 275 | 128 | 294 ppm | 72 ppm | 22 | 1.5 |
| OP'-BPA: 2-(2-Hydroxyphenyl)-2-(4-hydroxyphenyl)propane Ex: Example C. Ex: Comparative Example | | | | | | | |

### Example 43

A mixture of 3% water and acetone and phenol at a mol ratio of 1 : 5 was placed in a glass-made reactor and maintained at 50°C. In the state, a hydrogen chloride gas was blown in the mixture to initiate the reaction. After almost all of acetone was consumed by the reaction, the reaction mixture was distilled to remove the hydrogen chloride gas, after adding water such that the concentration became 3%, the reaction mixture was cooled to 45°C to precipitate adducts (the 1st crystallization), and the adducts obtained were separated from the mother liquor. The adducts obtained were dissolved in phenol, recrystallized at 45°C (the 2nd crystallization), the adducts obtained were heated to 180°C at a pressure of 40 Torr to remove phenol such that the phenol concentration in the bisphenol A became 3%, and further phenol was removed by steam stripping to obtain a bisphenol A. The contents of 9,9'-dimethylxanthene and iron in the bisphenol obtained are shown in Table 6.

The reaction of 510.8 g of the bisphenol A and 530.0 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain a polycarbonate. As the result thereof, the intrinsic viscosity [η] of the polycarbonate obtained was 0.35 and the b value showing the hue of the polymer was 0.1. The results are shown in Table 6.

### comparative Example 22

By following the same procedure as Example 3 except that the number of the crystallization was changed to once, a bisphenol A was obtained. The contents of 9,9'-dimethylxanthene and in the bisphenol obtained are shown in Table 6. Also, the content of 2,4'-bisphenol A which is an isomer is 270 ppm, which was almost same content in a commercially available bisphenol A. When a polycarbonate was obtained by the polymerization as Example 43 using the bisphenol A, the intrinsic viscosity of the polycarbonate obtained was 0.35 and the b value showing the hue of the polymer was 2.5. The results are shown in Table 6.

### Example 44

By following the same procedure as Example 43 except that 9, 9'-dimethylxanthene obtained from the mother liquor of the 1st crystallization obtained in Example 43 by the preparative chromatography was added to the bisphenol A obtained in Example 43 such that the content of 9,9'-dimethylxanthene in the bisphenol A became 1.6 ppm, an aromatic polycarbonate was obtained by the polymerization. The results are shown in Table 6.

### Comparative Example 23

By following the same procedure as Example 43 except that 9,9'-dimethylxanthene was added to the bisphenol A such that the content of 9,9'-dimethylxanthene in the bisphenol A became 3 ppm, an aromatic polycarbonate was obtained by the polymerization. The results thereof are shown in Table 6.

### Example 45

To the bisphenol A obtained in Example 43 was added an aqueous 0.01% iron(III) chloride solution such that the content of iron in the bisphenol became 70 ppb. After melting the bisphenol A and sufficiently stirring, diphenyl carbonate and tetramethylammonium hydroxide were added and the reaction was carried out as in Example 43. The results are shown in Table 6.

**Table 6**

| | Bisphenol A | | Polycarbonate property | |
|---|---|---|---|---|
| | DMX (ppm) | Fe (ppb) | Intrinsic viscosity | b Value |
| Example 43 | 0.3 | 10 | 0.35 | 0.1 |
| Comparative Example 22 | 4 | 60 | 0.35 | 2.5 |
| Example 44 | 1.6 | 10 | 0.36 | 0.8 |
| Comparative Example 23 | 3 | 10 | 0.35 | 1.5 |
| Example 45 | 0.3 | 60 | 0.36 | 1 |
| DMX: 9,9'-Dimethylxanthene | | | | |

### Example 46

Using a sulfonic acid-base ion-exchange resin of which 15% of the whole sulfonic acid groups was modified by cysteamine as a catalyst, acetone was reacted with phenol at mol ratio of 1 : 12 at a reaction temperature of 80°C to obtain a reaction mixture. The reaction mixture was cooled to 45°C to precipitate adducts (the 1st crystallization) and the adducts obtained were separated from the mother liquor. After purging 10% of the total amount of the mother liquor separated from the system, water was removed from the mother liquor and the mother liquor was recycled to the reactor. The state was continued until the concentrations of the organic impurities in the system became constant, when the concentrations of the organic impurities became constant, the adducts obtained were dissolved in phenol and the recrystallization was carried out at 45°C (the 2nd crystallization) to obtain adducts. The adducts obtained were further dissolved in phenol to carry out the 3rd crystallization, the adducts obtained were heated to 180°C at a pressure of 40 Torr to remove phenol until the phenol concentration in the bisphenol A became 3% and further phenol was removed by steam stripping to obtain a bisphenol A. The content of 2-(4-hydroxyphenyl)-2,4,4-trimethylchroman and the content of each element of the group A in the bisphenol A obtained are shown in Table 7.

The reaction of 102.8 g of the bisphenol A and 106.0 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain a polycarbonate. As the result thereof, the intrinsic viscosity [η] of the polycarbonate obtained was 0.38 and the b value showing the hue of the polymer was 0.1. The results are shown in Table 7.

### Comparative Example 24

By following the same procedure as Example 46 except that the number of the crystallization was changed to twice, a bisphenol A was obtained. The contents of 2-(4-hydroxyphenyl)-2,4,4-trimethylchroman and each element of group A in the bisphenol A obtained are show in Table 7. Also, the content of 2,4'-bisphenol A, which is an isomer, was 200 ppm which was almost same content as a commercially available bisphenol A. Furthermore, when a polycarbonate was obtained by the polymerization as in Example 46 using the bisphenol A, the intrinsic viscosity of the polycarbonate obtained was 0.37 and the b value thereof showing the hue of the polymer was 2.0. The results are shown in Table 7.

### Examples 47 and 48

By following the same procedure as Example 46 except that 2-(4-hydroxyphenyl)-2,4,4-trimethylchroman dispensed by dispense chromatography from the mother liquor of the 1st crystallization obtained in Example 46 was added to the bisphenol A obtained in Example 46 such that the content of 2-(4-hydroxyphenyl)-2,4,4-trimethylchroman in the bisphenol A became 25 ppm and 40 ppm respectively, aromatic polycarbonates were obtained by the polymerization. The results thereof are shown in Table 7.

### Comparative Example 25

By following the same procedure as Example 47 except that 2-(4-hydroxyphenyl)-2,4,4-trimethylchroman was added to the bisphenol A such that the content of 2-(4-hydroxyphenyl)-2,4,4-trimethylchroman in the bisphenol A became 60 ppm, an aromatic polycarbonate was obtained by the polymerization. The results thereof are shown in Table 7.

### Example 49

To the bisphenol A obtained in Example 46 was added an aqueous 0.01% sodium hydroxide solution such that the content of sodium in the bisphenol A became 90 ppb. After melting the bisphenol and sufficiently mixing, diphenyl carbonate and tetramethylammonium hydroxide were added thereto and the polymerization was carried out as in Example 46. The results thereof are shown in Table 7.

### Examples 50 to 54

By following the same procedure as Example 49 except that, in place of the aqueous 0.01% sodium hydroxide solution, an aqueous 0.01% iron (III) chloride solution, an aqueous 0.01% nickel (II) chloride solution, an aqueous 0.01% chromium (III) chloride solution, an aqueous manganese (II) chloride solution, or an aqueous lead (II) nitrate solution was added to the bisphenol A respectively such that the content of the element of the group A in the bisphenol A became 60 ppb, each polycarbonate was obtained. The results thereof are shown in Table 7.

### Example 55

By following the same procedure as Example 49 except that the content of sodium in the bisphenol A became 40 ppb, a polycarbonate was obtained. The results thereof are shown in Table 7.

### Examples 56 to 65

By following the same procedures as Examples 50 to 54 except that the addition amount of each aqueous solution containing the element of the group A was controlled such that the content of each element of the group A became 30 ppb and 15 ppb respectively, polycarbonates were obtained. The results thereof are shown in Table 7.

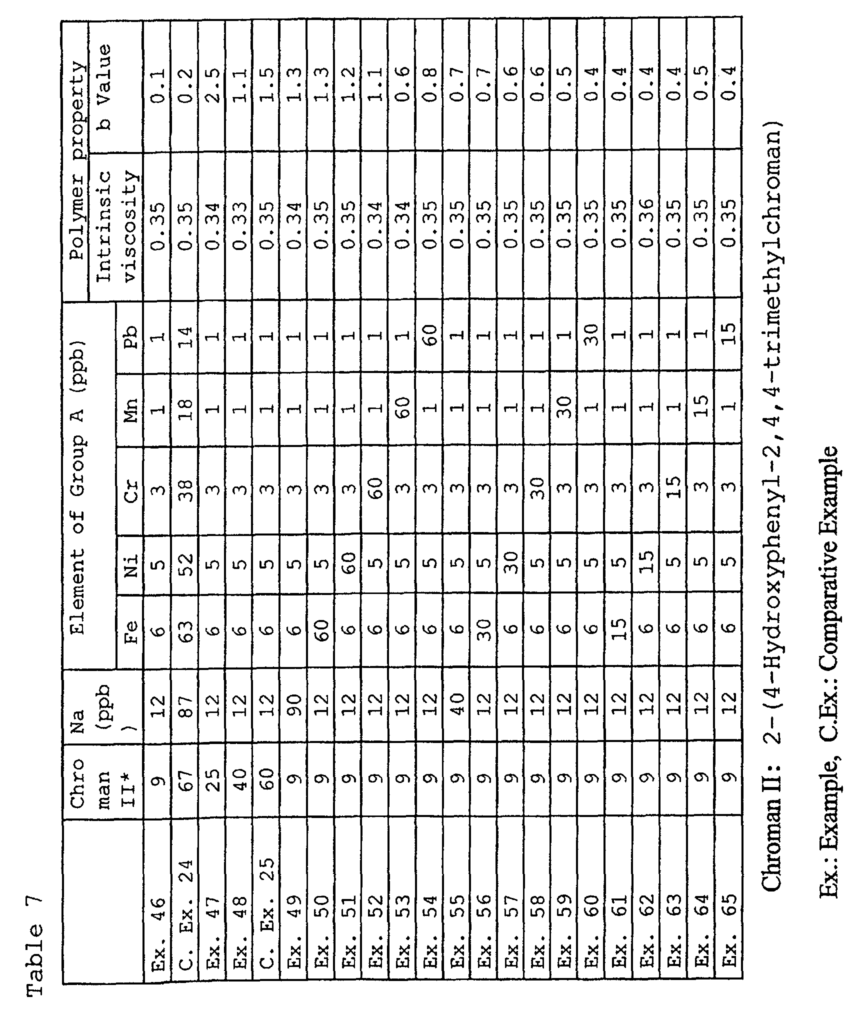

### Example 66

A mixture of 3% water and acetone and phenol at a mol ratio of 1 : 5 was placed in a glass-made reactor and maintained at 55°C. In the state, a hydrogen chloride gas was blown in the mixture to initiate the reaction. After almost all of acetone was consumed by the reaction, the reaction mixture was distilled to remove the hydrogen chloride gas, after adding water such that the concentration became 3%, the reaction mixture was cooled to 45°C to precipitate adducts (the 1st crystallization), and the adducts obtained were separated from the mother liquor. The adducts obtained were dissolved in phenol, recrystallized at 45°C (the 2nd crystallization), the adducts obtained were heated to 180°C at a pressure of 40 Torr to remove phenol such that the phenol concentration in the bisphenol A became 3%, and further phenol was removed by steam stripping to obtain a bisphenol A. The contents of 2,2,4-trimethylchromene and iron in the bisphenol A obtained are shown in Table 8.

The reaction of 510 g of the bisphenol A and 530 g of diphenyl carbonate was carried out with stirring using tetramethylammonium as the catalyst by reducing the pressure to 15 mmHg and increasing the temperature to 240°C. The reaction of the reaction product obtained was carried out in a thin-film type reactor at a pressure of 4 mmHg and a temperature of 260°C to obtain a prepolymer. The prepolymer was further polymerized at 280°C and a pressure of 0.5 mmHg to obtain a polycarbonate. As the result thereof, the intrinsic viscosity [η] of the polycarbonate obtained was 0.35 and the b value showing the hue of the polymer was 0.1. The results are shown in Table 8.

### Comparative Example 26

By following the same procedure as Example 66 except that the number of the crystallization was changed to once, a bisphenol A was obtained. The contents of 2,2,4-trimethylchromene and iron in the bisphenol A obtained are shown in Table 8. Also, the content of 2, 4'-bisphenol A, which is an isomer, was 290 ppm which was almost sale content of a commercially available bisphenol A. Furthermore, when a polycarbonate was obtained by the polymerization as in Example 66 using the bisphenol A, the intrinsic viscosity of the polycarbonate obtained was 0.35 and the b value showing the hue of the polymer was 2.5. The results are shown in Table 8.

### Example 67

By following the same procedure as Example 66 except that to the bisphenol A obtained in Example 66 was added 2,2,4-trimethylchromene dispensed by dispense chromatography from the mother liquor of the 1st crystallization obtained in Example 66 such that the content of 2,2,4-trimethylchromene in the bisphenol A became 3 ppm, an aromatic polycarbonate was obtained by the polymerization. The results thereof are shown in Table 8.

### comparative Example 27

By following the same procedure as Example 67 except that 2,2,4-trimethylchromene was added to the bisphenol A such that the content of 2,2,4-trimethylchromene in the bisphenol A became 7 ppm, an aromatic polycarbonate was obtained by the polymerization. The results thereof are shown in Table 8.

### Examples 68 and 69

An aqueous 0.01% iron (III) chloride solution was added to the bisphenol A obtained in Example 66 such that the content of iron in the bisphenol A became 20 ppb and 70 ppb respectively. After melting each bisphenol A and sufficiently mixing, diphenyl carbonate and tetramethylammonium hydroxide were added to each bisphenol A and the polymerization was carried out as in Example 66. The results thereof are shown in Table 8 below.

**Table 8**

| | Bisphenol A | | Polycarbonate property | |
|---|---|---|---|---|
| | Chromene (ppm) | Fe (ppb) | Intrinsic viscosity | b Value |
| Example 66 | 1 | 10 | 0.35 | 0.1 |
| Comparative Example 26 | 7 | 60 | 0.35 | 2.5 |
| Example 67 | 3 | 10 | 0.35 | 0.7 |
| Comparative Example 27 | 7 | 10 | 0.36 | 1.5 |
| Example 68 | 1 | 20 | 0.35 | 0.4 |
| Example 69 | 1 | 70 | 0.36 | 1 |
| Chromene: 2,2,4-trimethylchromene | | | | |

### INDUSTRIAL APPLICABILITY

The high-quality bisphenol A gives less coloring of the aromatic polycarbonate prepared using the bisphenol A as the raw material and molded, and particularly, is useful as raw materials of optical uses such as optical disks, etc., severely required for the transparency and the hue.

## Claims

1. A high-quality bisphenol A selected from the following groups (a) to (g);
(a) A high-quality bisphenol A wherein content of sodium is not more than 80 ppb and the content of each element of group A consisting of iron, nickel, chromium, manganese, lead, and molybdenum is not more than 50 ppb.
(b) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 15.5 minutes to 24 minutes is not more than 2.0 × 10⁻³ as the rate to the absorption peak area of the bisphenol A.
(c) A high-quality bisphenol A wherein a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 22 minutes to 24 minutes is not more than 50 ppm to the area of the absorption peak of the bisphenol A.
(d) A high-quality bisphenol A wherein the sum total of the contents of the 1-naphthol derivatives represented by following formula (1) is not more than 200 ppm to the bisphenol A; 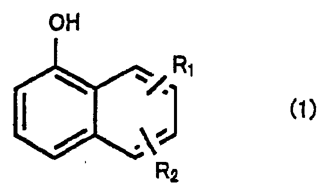 wherein R₁ and R₂ each represents a methyl group, an ethyl group, an n-propyl group, an isopropyl group, or an isopropenyl group each bonded to the 2 to 8 position of the 1-naphthol.
(e) A high-quality bisphenol A wherein the content of 9,9'-dimethylxanthene is not more than 2 ppm.
(f) A high-quality bisphenol A wherein the content of 2-(4-hydroxyphenyl)-2,4,4-trimethylchroman is not more than 50 ppm.
(g) A high-quality bisphenol A wherein the content of 2,2,4-trimethylchromene is not more than 5 ppm.

2. The high-quality bisphenol A according to claim 1 wherein the high-quality bisphenol A is (a) and the content of each element of group B consisting of copper, zinc, aluminum, and indium is not more than 20 ppb.

3. The high-quality bisphenol A according to claim 1 wherein the high-quality bisphenol A is (a) and the content of titanium is not more than 1 ppb.

4. The high-quality bisphenol A according to claim 1 wherein the high-quality bisphenol A is (a) and the content of each element of the group A is not more than 20 ppb.

5. The high-quality bisphenol A according to claim 1 wherein the high-quality bisphenol A is (a) and the content of each element of the group A is not more than 10 ppb.

6. The high-quality bisphenol A according to claim 1 wherein the high-quality bisphenol A is (a) and the content of silicon is not more than 20 ppb, with the proviso that lead is excluded from the elements of the group A.

7. The high-quality bisphenol A according to claim 1 wherein the high-quality bisphenol A is (b) and when the bisphenol A is analyzed by a ultraviolet detector of a wavelength of 254 nm, the sum total of the absorption peak areas of the compounds eluted between the time of from 15.5 minutes to 24 minutes is not more than 1.0 × 10⁻³ to the area of the absorption peak of the bisphenol A.

8. The high-quality bisphenol A according to claim 1 wherein the high-quality bisphenol A is (b), (c), (d), (e) or (g), and the content of iron is not more than 50 ppb.

9. The high-quality bisphenol A according to claim 1 wherein the high-quality bisphenol A is (c), and when the bisphenol is analyzed by a ultraviolet detector of a wavelength of 254 nm, the sum total of the absorption peak areas of the compounds having molecule weights of from 307 to 309 eluted between the time of from 22 minutes to 24 minutes is not more than 20 ppm to the area of the absorption peak of the bisphenol A.

10. The high-quality bisphenol A according to claim 1 wherein the high-quality bisphenol A is (d) and the sum total of the contents of 1-naphthol derivatives is not more than 100 ppm.

11. The high-quality bisphenol A according to claim 1 wherein the high-quality bisphenol A is (d) and a measurement is carried out by a high performance liquid chromatography in which a stainless steel-made column having an inner diameter of 4.6 mm and a length of 250 mm packed with an adsorbent obtained by bonding an octadecyl group to a high-pure spherical silica gel having a pore diameter of 100 angstroms in an amount of 15% of the silica gel is maintained at 40°C, using an aqueous 0.1% phosphoric acid solution and acetonitrile as an eluent A and an eluent B respectively such that from the initiation of the measurement to 5 minutes after the initiation of the measurement, the ratio of the eluent A : the eluent B is 1 : 1 and the flow rate of the sum total of the eluent A and the eluent B becomes 0.9 ml/minute, while keeping the flow rate of the sum total at constant, a gradient operation of increasing the rate of the eluent B from 5 minutes after the initiation of the measurement and then increasing the rate of the eluent B such that the ratio of the eluent A : the eluent B becomes 0 : 1 until 55 minutes after the initiation of the measurement is carried out, and then when the bisphenol A is analyzed by a ultraviolet detector of a wavelength of 254 nm, the sum total of the absorption peak areas of compounds eluted between the time of from 22 minutes to 24 minutes is not more than 50 ppm to the area of the absorption peak of the bisphenol A.

12. The high-quality bisphenol A according to claim 1 wherein the high-quality bisphenol A is (f), the content of sodium is not more than 80 ppb, and the content of each element of the group A consisting of iron, nickel, chromium, manganese, lead, and molybdenum is not more than 50 ppb.

13. The high-quality bisphenol A according to claim 1 wherein the high-quality bisphenol A is (f) and the content of 2-(4-hydroxyphenyl-2,4,4-trimethylchroman is not more than 30 ppm.

14. The high-quality bisphenol A according to claim 12 wherein the content of each element of the group A is not more than 20 ppb.

15. The high-quality bisphenol A according to claim 12 wherein the content of each element of the group A is not more than 10 ppb.

16. The high-quality bisphenol A according to any one of claims 1 to 15 wherein the bisphenol A is a raw material of an aromatic polycarbonate for optical uses.

17. In a process of preparing a bisphenol A by reacting phenol and acetone,
the improved process of preparing a high-quality bisphenol A wherein the content of sodium is not more than 80 ppb and the content of each element of the group A consisting of iron, nickel, chromium, manganese, lead, and molybdenum is not more than 50 ppb, which comprises contacting the reaction mixture obtained from the reaction of phenol and acetone with an absorbent.

18. In a process of forming a bisphenol A by reacting phenol and acetone in a reactor, and separating equimolar adducts of the bisphenol A and phenol and a mother liquor containing phenol from the reaction mixture obtained,
the improved process for preparing a high-quality bisphenol A wherein the content of sodium is not more than 80 ppb and the content of each element of the group A consisting of iron, nickel, chromium, manganese, lead, and molybdenum is not more than 50 ppb, which comprises after contacting the mother liquor with an absorbent, recycling the mother liquor to the reactor.
